# EUROPÄISCHE PATENTSCHRIFT

(11) **EP 3 169 276 B1**
(45) Veröffentlichungstag und Bekanntmachung des Hinweises auf die Patenterteilung: **14.10.2020**
(21) Anmeldenummer: 14741272.0
(22) Anmeldetag: 16.07.2014
(51) Int. Cl.: A61F 2/24

(54) **HERZKLAPPENPROTHESE ZUM PERKUTANEN ERSATZ EINER TRIKUSPIDALKLAPPE UND SYSTEM MIT EINER DERARTIGEN HERZKLAPPENPROTHESE**
HEART VALVE PROSTHESIS FOR PERCUTANEOUS REPLACEMENT OF A TRICUSPID VALVE, AND SYSTEM COMPRISING A HEART VALVE PROSTHESIS OF SAID TYPE
PROTHÈSE DE VALVULE CARDIAQUE PERMETTANT LE REMPLACEMENT PERCUTANÉ DE LA VALVULE TRICUSPIDE ET SYSTÈME COMPORTANT UNE TELLE PROTHÈSE DE VALVULE CARDIAQUE

(43) Veröffentlichungstag der Anmeldung: 24.05.2017
(73) Patentinhaber: MEDIRA AG, 5630 Muri AG (CH)
(72) Erfinder: FIGULLA, Hans Reiner, 07749 Jena (DE); LAUTEN, Alexander, 14532 Kleinmachnow (DE)
(74) Vertreter: Witte, Weller & Partner Patentanwälte mbB
(86) Internationale Anmeldenummer: PCT/EP2014/065294
(87) Internationale Veröffentlichungsnummer: WO 2016/008526

(56) Entgegenhaltungen:
- EP-A1- 2 929 860
- US-A1- 2006 276 813
- US-A1- 2007 156 233
- US-A1- 2009 264 991
- US-A1- 2012 136 430
- US-A1- 2013 261 739

## Beschreibung

Die Erfindung betrifft eine Herzklappenprothese zum perkutanen Ersatz einer Trikuspialklappe. Ferner befasst sich die Erfindung mit einem System mit einer derartigen Herzklappenprothese.

Aus medizinischer Sicht stellt die operative Behandlung der Trikuspidalklappeninsuffizienz eine hohe Herausforderung dar. Dabei lässt die Dichtigkeit der Trikuspidalklappe, die den rechten Vorhof bzw. das rechte Atrium des menschlichen Herzens von der rechten Herzkammer bzw. dem rechten Ventrikel trennt, nach. Dies kann dazu führen, dass aufgrund der Kontraktion des rechten Ventrikels Blutvolumen in die obere und/oder untere Hohlvene zurückgedrängt wird. Dies führt schlussendlich zu einer Volumenbelastung des rechten Herzens sowie zu einer Druckerhöhung im venösen Kreislaufsystem. In fortgeschrittenen Stadien kann es zu einem Rechtsherzversagen mit Leberstauung und Ödembildung in peripheren Blutgefäßen kommen.

Je nach Schweregrad der Erkrankung ist es erforderlich, die natürliche Trikuspidalklappe vollständig durch eine künstliche Herzklappe zu ersetzen. Derartige chirurgische Eingriffe sind äußerst aufwändig und führen zu einer hohen Belastung des Patienten. Das bisherige Operationsverfahren wird am offenen Herzen durchgeführt, so dass der Patient während der Operation an eine Herz-Lungen-Maschine angeschlossen werden muss. Dies erhöht die Komplexität des Eingriffs und die Kreislaufbelastung für den Patienten. Entsprechend hoch ist die Mortalität bei solchen Eingriffen. Es besteht daher ein Bedürfnis nach einer minimalinvasiven Möglichkeit zur Therapie der Trikuspidalklappeninsuffizienz. Beispielsweise offenbart die US 2006/0276813 einen T-förmigen Stent, welcher innerhalb des rechten Vorhofs platziert wird. Die Fixierung der Prothese innerhalb des rechten Vorhofs wird dabei durch drei Gerüststrukturen erreicht, die jeweils innerhalb der oberen und unteren Hohlvene und der Trikuspidalklappe expandiert werden und so den rechten Vorhof größtenteils durch den Stent auskleiden. Eine weitere Herzklappenprothese wird durch die EP 2 929 860 A1 offenbart. Diese ähnelt dem T-förmigen Stent gemäß der US 2006/0276813; sie ist jedoch zweiteilig aufgebaut, wobei ein erster Stent im implantierten Zustand innerhalb der oberen und unteren Hohlvene fixiert ist und der zweite Stent im Bereich des rechten Vorhofs am ersten Stent fixiert wird.

Die Aufgabe der Erfindung besteht darin, eine Herzklappenprothese anzugeben, die zum perkutanen Ersatz einer Trikuspidalklappe geeignet ist und sich einfach minimalinvasiv chirurgisch implantieren lässt. Ferner besteht die Aufgabe der Erfindung darin, ein System mit einer derartigen Herzklappenprothese anzugeben.

Erfindungsgemäß wird diese Aufgabe im Hinblick auf die Herzklappenprothese durch den Gegenstand des Patentanspruchs 1 und im Hinblick auf das System durch den Gegenstand des Patentanspruchs 12 gelöst.

Die Erfindung beruht auf dem Gedanken, eine Herzklappenprothese zum perkutanen Ersatz einer Trikuspidalklappe mit einer stentartigen Tragstruktur und einer biologischen Herzklappe anzugeben, wobei die stentartige Tragstruktur von einem radial komprimierten in einen radial expandierten Zustand selbsttätig expandierbar ist und eine fluiddichte Umhüllung aufweist. Die fluiddichte Umhüllung erstreckt sich zumindest abschnittsweise rohrförmig über die Tragstruktur. Dabei ist die Herzklappe in einer seitlichen Öffnung der Tragstruktur angeordnet.

Die Erfindung beruht auf der Idee, den aufwändigen operativen Eingriff zum vollständigen Austausch bzw. zur operativen Korrektur der Trikuspidalklappe dadurch zu umgehen, dass zusätzlich zur bestehenden Trikuspidalklappe eine Herzklappenprothese minimalinvasiv eingesetzt wird . Die Herzklappenprothese, insbesondere deren biologische Herzklappe, ersetzt dabei zumindest teilweise die Funktion der natürlichen Trikuspidalklappe. Der Vorteil der erfindungsgemäßen Herzklappe besteht darin, dass sie einen heterotopen, kathetergeführten Trikuspidalklappenersatz ermöglicht. Mit anderen Worten eignet sich die erfindungsgemäße Herzklappenprothese für eine minimalinvasive chirurgische Implantation. Dies reduziert die während der Operation auf den Patienten einwirkenden Belastungen und senkt das Mortalitätsrisiko.

Die erfindungsgemäße Herzklappenprothese ist insbesondere wegen der stentartigen Tragstruktur in einen radial komprimierten Zustand überführbar und kann so mittels eines Katheters beispielsweise über die Femoralvene transluminal an den Implantationsort geführt werden. Eine Operation am offenen Herzen kann so ggf. vermieden werden.

Die Herzklappenprothese spannt sich nach Entlassung aus dem Katheter selbsttätig am Implantationsort auf. Wegen der selbstexpandierenden Eigenschaften der Tragstruktur wird auf die Gefäßwände am Implantationsort ein Radialdruck ausgeübt, der zu einer Fixierung der Herzklappenprothese am Implantationsort führt. Gleichzeitig dichtet die Herzklappenprothese gut gegen die natürlichen Gefäßwände ab.

Die mit der fluiddichten Umhüllung versehene Tragstruktur ermöglicht es, den rechten Vorhof des menschlichen Herzens durch die Herzklappenprothese zu überbrücken und so sowohl die obere Hohlvene, als auch die untere Hohlvene zu verbinden. Im implantierten Zustand ist die Herzklappenprothese vorzugsweise einerseits in der oberen Hohlvene und andererseits in der unteren Hohlvene verankert und erstreckt sich insofern quer durch den rechten Herzvorhof.

Die seitlich angeordnete biologische Herzklappe ist vorzugsweise zur Trikuspidalklappe bzw. zum rechten Herzventrikel gerichtet und ermöglicht so den Einstrom von venösem Blut aus den Hohlvenen in den rechten Vorhof bzw. den rechten Ventrikel. Insofern ist die biologische Herzklappe der erfindungsgemäßen Herzklappenprothese in Serie zur natürlichen Trikuspidalklappe geschaltet. Dabei übernimmt die biologische Herzklappe der Herzklappenprothese die Funktion der natürlichen Trikuspidalklappe bzw. kompensiert deren Undichtigkeit. Insofern bietet die Erfindung eine einfache und effiziente Möglichkeit zur Therapie der Trikuspidalklappeninsuffizienz.

Bei der erfindungsgemäßen Herzklappenprothese ist die seitliche Öffnung in einer gemeinsamen Wandungsebene mit der stentartigen, rohrförmigen, Tragstruktur ausgebildet. Im Allgemeinen ist vorgesehen, dass die Herzklappenprothese insgesamt rohrförmig ausgebildet ist. Die fluiddichte Umhüllung der Tragstruktur gewährleistet eine Leitungsfunktion für einströmendes Blut und verhindert ein unkontrolliertes Abströmen von Blut in den rechten Herzvorhof. Insofern bildet die Tragstruktur, insbesondere gemeinsam mit der Umhüllung, eine rohrförmige Wandung der Herzklappenprothese.

Die seitliche Öffnung der Tragstruktur ist in einer Ebene mit der rohrförmigen Wandung angeordnet. Die seitliche Öffnung folgt also der Krümmung der rohrförmigen Wandung bzw. dem Verlauf der gemeinsamen Wandungsebene. Vorzugsweise erstreckt sich die seitliche Öffnung nicht nur durch die Tragstruktur, sondern auch durch die Umhüllung und bildet insofern einen Zugang zum Innenlumen der Herzklappenprothese.

In der seitlichen Öffnung ist die biologische Herzklappe angeordnet. Mit anderen Worten ist die seitliche Öffnung durch die biologische Herzklappe verschlossen. Die biologische Herzklappe übernimmt eine Ventilfunktion. Insbesondere verhindert die biologische Herzklappe ein Einströmen von Blut aus dem rechten Vorhof in die Herzklappenprothese. Dabei kann die biologische Herzklappe als Rückschlagventil wirken, das einen Blutstrom ausschließlich von den Hohlvenen in den rechten Vorhof ermöglicht und einen Rückstrom verhindert.

Bei einer weiteren bevorzugten Ausführungsform der erfindungsgemäßen Herzklappenprothese ist vorgesehen, dass die Tragstruktur zwei längsaxiale Endabschnitte aufweist, die zur Verankerung in der oberen und unteren Hohlvene angepasst sind. Die Herzklappenprothese ist im Allgemeinen derart angepasst, dass sie die obere Hohlvene und die untere Hohlvene miteinander verbinden kann. Die Länge der Herzklappenprothese ist daher so gewählt, dass die längsaxialen Endabschnitte der Tragstruktur in Mündungsabschnitte der Hohlvenen eingreifen können, die einen Übergangsbereich zum rechten Vorhof bilden. Insofern kann die Herzklappenprothese eine Rohrverbindung zwischen der oberen Hohlvene und der unteren Hohlvene bilden. Dabei sind die längsaxialen Endabschnitte, insbesondere hinsichtlich ihres Querschnittsdurchmessers, so dimensioniert, dass sie mit einer ausreichenden Radialkraft gegen die Gefäßwände der Hohlvenen drängen und die Herzklappenprothese so am Implantationsort verankern.

Ferner kann bei der erfindungsgemäßen Herzklappenprothese vorgesehen sein, dass die Tragstruktur einen Atriumabschnitt aufweist, der sich zwischen den längsaxialen Endabschnitten erstreckt. Der Atriumabschnitt ist im implantierten Zustand vorzugsweise frei im rechten Vorhof angeordnet und wird durch die längsaxialen Endabschnitte in Position gehalten. Der Atriumabschnitt hat daher hauptsächlich eine Blutführungsfunktion, wogegen die längsaxialen Endabschnitte hauptsächlich eine Verankerungsfunktion aufweisen.

Um seiner Blutführungsfunktion gerecht zu werden, weist der Atriumabschnitt vorzugsweise eine Länge auf, die wenigstens dem Abstand zwischen der oberen Hohlvene und der unteren Hohlvene entspricht. Dies gewährleistet, dass der Atriumabschnitt eine Fluidverbindung zwischen der oberen Hohlvene und der unteren Hohlvene bzw. zwischen den Hohlvenen und der seitlichen Öffnung der Tragstruktur, bereitstellt. Insofern ist vorgesehen, dass die seitliche Öffnung, insbesondere die Herzklappe, im Atriumabschnitt der Tragstruktur angeordnet ist.

In diesem Zusammenhang wird darauf hingewiesen, dass die Rohrform der Herzklappenprothese nicht zwingend voraussetzt, dass die Herzklappenprothese einen konstanten Querschnittsdurchmesser aufweist. Es ist vielmehr auch möglich, dass entlang der Herzklappenprothese bzw. der stentartigen Tragstruktur deren Querschnittsdurchmesser variiert. Beispielsweise können die längsaxialen Endabschnitte einen größeren oder kleineren Querschnittsdurchmesser als der dazwischenliegende Atriumabschnitt aufweisen.

Ferner wird an dieser Stelle darauf hingewiesen, dass in der vorliegenden Anmeldung die Form der Herzklappenprothese, insbesondere der stentartigen Tragstruktur, im vollständig expandierten Zustand beschrieben wird. Im vollständig expandierten Zustand wirken auf die Tragstruktur bzw. die Herzklappenprothese keine äußeren Kräfte, die die Tragstruktur beispielsweise radial komprimieren könnten. Der vollständig expandierte Zustand entspricht daher im Wesentlichen einem Ruhezustand der Herzklappenprothese. Im implantierten Zustand nimmt die Herzklappenprothese zumindest abschnittsweise meist eine andere Kontur bzw. Form ein, da die radiale Expansion der Tragstruktur, insbesondere im Bereich der längsaxialen Endabschnitte, durch Kontakt mit Körpergewebe bzw. mit den Gefäßwänden begrenzt wird.

Die Tragstruktur kann im Allgemeinen ein Drahtgeflecht und/oder eine lasergeschnittene Gitterstruktur aufweisen, die jeweils geschlossene Maschen oder Zellen umfassen. Das Drahtgeflecht bzw. die Gitterstruktur können mit der Umhüllung versehen sein, wobei die Umhüllung sich sowohl auf einen Außenumfang, als auch auf einen Innenumfang der Gitterstruktur bzw. des Drahtgeflechts erstrecken kann. Es ist auch möglich, dass sowohl auf einem Innenumfang, als auch auf einem Außenumfang der Gitterstruktur bzw. des Drahtgeflechts eine Umhüllung angeordnet ist.

Die Verwendung eines Drahtgeflechts bzw. einer lasergeschnittenen Gitterstruktur mit geschlossenen Zellen hat mehrere Vorteile. Einerseits ermöglicht eine derartige Tragstruktur eine gute Komprimierung, so dass sich die Herzklappenprothese gut über einen Katheter an den Implantationsort führen lässt. Gleichzeitig ist das Durchmesserverhältnis zwischen komprimierten Zustand und expandierten Zustand ausreichend groß, so dass die Tragstruktur mit einer geeigneten Radialkraft in den Hohlvenen verankert werden kann. Überdies stellt eine Gitterstruktur bzw. ein Drahtgeflecht mit geschlossenen Zellen oder Maschen sicher, dass die Tragstruktur in einen Katheter wieder zurückgezogen werden kann, beispielsweise um eine Fehlpositionierung zu korrigieren. Schließlich ist die Stabilität von Gitterstrukturen bzw. Drahtgeflechten mit geschlossenen Zellen bzw. Maschen zweckmäßig für die erfindungsgemäß vorgesehene Anwendung.

Die Tragstruktur kann außerdem an wenigstens einem längsaxialen Ende ein Durchlasssegment aufweisen, das frei von der Umhüllung ist. Das Durchlasssegment kann insbesondere ein Ring von in Umfangsrichtung der Tragstruktur benachbart angeordneten Maschen oder Zellen sein, die, insbesondere wegen der fehlenden Umhüllung, blutdurchlässig sind. Vorzugsweise ist das Durchlasssegment an einem längsaxialen Ende der Tragstruktur angeordnet. Im Bereich der unteren Hohlvene münden kurz vor dem Eintritt in den rechten Vorhof mehrere Lebervenen.

Um die Blutzufuhr aus den Lebervenen in die untere Hohlvene weiterhin zu ermöglichen, kann das in der unteren Hohlvene zu platzierende längsaxiale Ende das Durchlasssegment aufweisen. Das Durchlasssegment ist daher vorzugsweise so angeordnet, dass es im implantierten Zustand im Bereich des Zuflusses der Lebervenen positionierbar ist. So kann das längsaxiale Ende der Tragstruktur einerseits eine ausreichende Fixierungsfunktion zur Verankerung der Herzklappenprothese in der unteren Hohlvene bereitstellen und gewährleistet dennoch den Zustrom von Blut aus den Lebervenen in die untere Hohlvene.

Um den Zustrom von Blut aus den Lebervenen in die untere Hohlvene verbessert aufrechtzuerhalten, kann außerdem vorgesehen sein, dass das Durchlasssegment Maschen bzw. Zellen mit einer Maschenweite aufweist, die größer als die Maschenweite anderer Maschen der Tragstruktur ist. Dies erhöht die Durchtrittsfläche für durchströmendes Blut und reduziert die Gefahr, dass sich an der Tragstruktur Thromben bilden, die zu einem Verschluss der Lebervenen führen könnten.

Bei der vorliegenden Erfindung ist vorgesehen, dass die Herzklappe innerhalb der seitlichen Öffnung fest mit der Tragstruktur verbunden ist. Insbesondere kann die Herzklappe innerhalb der seitlichen Öffnung fest mit der Gitterstruktur oder dem Drahtgeflecht der Tragstruktur verbunden sein. Beispielsweise kann die Herzklappe mit der Tragstruktur vernäht sein. Die feste Verbindung mit der Tragstruktur, insbesondere dem Traggeflecht oder der Gitterstruktur, gewährleistet eine sichere Funktion der biologischen Herzklappe.

Ferner ist in bevorzugter Weise bei der Erfindung vorgesehen, dass sich die Umhüllung fluiddicht an die biologische Herzklappe anschließt. Im Allgemeinen ist vorgesehen, dass zumindest der Atriumabschnitt der Tragstruktur vollständig abgedichtet ist, wobei die Abdichtung durch die Umhüllung erfolgt. Lediglich die seitliche Öffnung, die im Atriumabschnitt angeordnet ist, ist umhüllungsfrei, wobei diese Lücke in der Umhüllung durch die biologische Herzklappe gefüllt wird. Mit Ausnahme der seitlichen Öffnung erstreckt sich die Umhüllung also vorzugsweise über die gesamte Länge und den gesamten Umfang zumindest des Atriumabschnitts der Tragstruktur.

Die längsaxialen Endabschnitte der Tragstruktur können umhüllungsfrei ausgebildet sein. Es ist jedoch auch möglich, dass sich die Umhüllung über die gesamte Tragstruktur erstreckt und lediglich im Bereich der seitlichen Öffnung frei ist. Dabei schließt die Umhüllung sich jedoch fluiddicht an die in der seitlichen Öffnung fixierte Herzklappe an, um eine ausreichende Abdichtung der biologischen Herzklappe sicherzustellen. Um eine ausreichende Dichtigkeit zu gewährleisten, kann die Umhüllung aus Polyesterfasern, beispielsweise Dacron-Fasern, gebildet sein.

Die Tragstruktur kann ein Formgedächtnismaterial und/oder ein superelastisches Material aufweisen. Insbesondere kann die Tragstruktur eine Nickel-Titan- Legierung, vorzugsweise Nitinol, aufweisen. Vorzugsweise besteht die Gitterstruktur bzw. das Drahtgeflecht aus einer solchen Nickel-Titan-Legierung. Derartige Formgedächtnismaterialien ermöglichen eine besonders gute selbstexpandierende Funktion und weisen die notwendige Biokompatibilität zur Implantation in Blutgefäßen auf. Ferner umfassen derartige Materialien eine ausreichend hohe Stabilität, so dass die stentartige Tragstruktur vergleichsweise dünn ausgebildet werden kann. Schließlich ermöglichen insbesondere Nickel- Titan-Legierungen eine gute Komprimierung der Tragstruktur, was die Implantation über einen Katheter erleichtert.

Insofern ist bevorzugt vorgesehen, dass die Herzklappenprothese im Allgemeinen transluminal, insbesondere transfemoral, implantierbar ist. Dies gewährleistet einen minimalinvasiven Eingriff und reduziert die Belastung des Patienten während der Operation. So kann die Herzklappenprothese über einen Katheter an den Behandlungsort geführt werden, wobei der Katheter vorzugsweise über die Femoralvene und die untere Hohlvene zum rechten Herzvorhof geschoben wird.

Alternativ kann ein Zugang über die Schlüsselbeinvene (vena subclavia) gewählt werden. Dabei wird die Herzklappenprothese über die obere Hohlvene zum rechten Herzvorhof geschoben. In beiden Fällen wird die Herzklappenprothese vorzugsweise sowohl in der oberen, als der unteren Hohlvene verankert, wobei die Verankerung durch die längsaxialen Endabschnitte der Tragstruktur erfolgt. Der Atriumabschnitt ist dann im rechten Vorhof angeordnet und bildet insofern eine Brücke zwischen der oberen Hohlvene und der unteren Hohlvene.

Gemäß einem nebengeordneten Aspekt beruht die Erfindung auf dem Gedanken, ein System mit einer zuvor beschriebenen Herzklappenprothese und einem Katheter anzugeben, wobei die Herzklappenprothese in einem komprimierten Zustand innerhalb des Katheters positionierbar und mittels des Katheters perkutan, insbesondere transluminal, vorzugsweise transfemoral, implantierbar ist.

Die Erfindung wird im Folgenden anhand eines Ausführungsbeispiels unter Bezugnahme auf die beigefügte, schematische Zeichnung näher erläutert. Darin zeigt die einzige Figur eine Seitenansicht einer erfindungsgemäßen Herzklappenprothese nach einem bevorzugten Ausführungsbeispiel im implantierten Zustand.

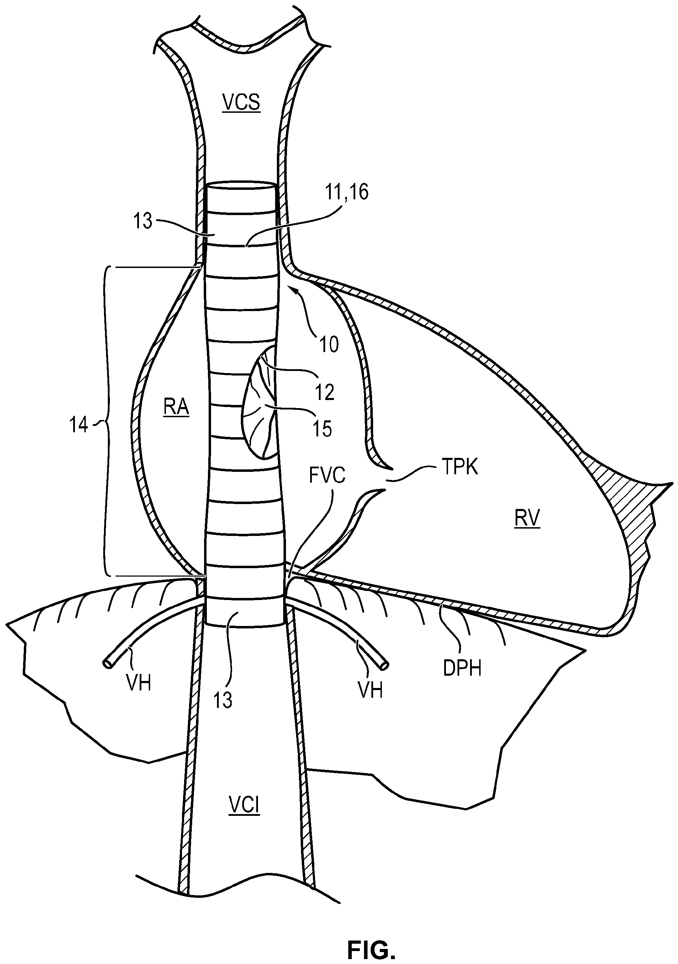

Fig. 1 zeigt den Bereich des rechten Herzens mit einem rechten Vorhof RA bzw. rechtem Atrium und einer rechten Herzkammer RV bzw. rechten Ventrikel. Zwischen dem rechten Vorhof RA und der rechten Kammer RV ist die natürliche Trikuspidalklappe TPK angeordnet. In den rechten Vorhof RA mündet einerseits die obere Hohlvene VCS und andererseits die untere Hohlvene VCI. Dabei erstreckt sich die untere Hohlvene VCI durch eine Zwerchfellöffnung FVC, die im Zwerchfell DPH angeordnet ist. Im Bereich der Zwerchfellöffnung FVC münden mehrere Lebervenen VH in die untere Hohlvene VCI.

Um in Folge einer Insuffizienz der Trikuspidalklappe TPK deren Funktion zumindest teilweise zu ersetzen, ist eine Herzklappenprothese 10 vorgesehen, die im Bereich des rechten Vorhofs RA implantiert wird. Die Herzklappenprothese 10 umfasst eine Tragstruktur 11 mit einer fluiddichten Umhüllung 16. Die Tragstruktur 11 bildet mit der fluiddichten Umhüllung 16 im Wesentlichen ein rohrförmiges bzw. schlauchförmiges Implantat, das sich von der oberen Hohlvene VCS zur unteren Hohlvene VCI erstreckt.

Die Tragstruktur 11 ist in drei Abschnitte unterteilbar, wobei zwei längsaxiale Endabschnitte 13 einen mittleren Atriumabschnitt 14 begrenzen. Die längsaxialen Endabschnitte 13 sind in den Hohlvenen VCS, VCI angeordnet und verankern die Herzklappenprothese 10 so am Implantationsort. Dazu ist vorgesehen, dass die Tragstruktur 11 bzw. insgesamt die Herzklappenprothese 10 selbsttätig expandierbar ist und bei der Expansion eine Radialkraft ausübt, die eine sichere Verankerung der Herzklappenprothese 10 in den Hohlvenen VCS, VCI gewährleistet.

Der Atriumabschnitt 14 der Tragstruktur 11 umfasst eine seitliche Öffnung 12, die in dem dargestellten Ausführungsbeispiel zur natürlichen Trikuspidalklappe TPK hin ausgerichtet ist. Die seitliche Öffnung 12 weist im Wesentlichen eine runde bzw. ovale Form auf und folgt der rohrförmigen Kontur des Atriumabschnitts 14. In der seitlichen Öffnung 12 ist eine biologische Herzklappe 15 angeordnet. Die biologische Herzklappe 15 ist mit der Umhüllung 16 der Tragstruktur 11 fluiddicht verbunden, so dass Blut, welches aus den Hohlvenen VCS, VCI in die Herzklappenprothese 10 einströmt, ausschließlich über die biologische Herzklappe 15 in den rechten Herzvorhof RA gelangen kann.

Im Bereich der unteren Hohlvene VCI kann der längsaxiale Endabschnitt 13 der Tragstruktur 11 außerdem ein Durchlasssegment (nicht dargestellt) aufweisen. Das Durchlasssegment kann umhüllungsfrei sein, so dass ein Zustrom von venösem Blut aus den Lebervenen VH in die untere Hohlvene VCI ermöglicht ist.

Im Allgemeinen zeigt die beigefügte Figur also eine erfindungsgemäße Herzklappenprothese 10, bei der es sich insbesondere um eine eine biologische Herzklappe tragende, gecoverte, selbstexpandierbare Rohrprothese handelt. Die Umhüllung 16 bzw. das Covering, kann durch eine Textilstruktur, beispielsweise aus Polyesterfasern, insbesondere aus Dacron-Fasern, gebildet sein. Die Herzklappenprothese 10 ist vorzugsweise kathetergeführt von transfemoral an den Implantationsort führbar. Dabei wird die Herzklappenprothese 10 vorzugsweise so im rechten Vorhof RA implantiert, dass die obere und untere Hohlvene VCS, VCI, durch die Herzklappenprothese 10 miteinander verbunden werden.

Die biologische Herzklappe 15, die in der seitlichen Öffnung 12 angeordnet ist, ist vorzugsweise so in den Atriumabschnitt 14 der Tragstruktur 11 integriert, dass die biologische Herzklappe 15 im implantierten Zustand auf Höhe der natürlichen Trikuspidalklappe TPK positionierbar ist. Insofern übernimmt die biologische Herzklappe 15 die Funktion der insuffizienten bzw. undichten natürlichen Trikuspidalklappe TPK. Im implantierten Zustand ermöglicht die erfindungsgemäße Herzklappenprothese 10, dass der Blutstrom aus der oberen Hohlvene VCS und der unteren Hohlvene VCI über die rohrförmige Herzklappenprothese 10 und die seitlich positionierte biologische Herzklappe in den rechten Vorhof RA geführt wird.

## Patentansprüche

1. Herzklappenprothese (10) zum Behandeln einer insuffizienten Trikuspidalklappe (TPK) mit einer stentartigen rohrförmigen Tragstruktur (11) und einer biologischen Herzklappe (15), wobei die stentartige Tragstruktur (11) von einem radial komprimierten in einen radial expandierten Zustand selbsttätig expandierbar ist und eine fluiddichte Umhüllung (16) aufweist, die sich zumindest abschnittsweise rohrförmig über die Tragstruktur (11) erstreckt, wobei die Herzklappe (15) in einer seitlichen Öffnung (12) der Tragstruktur (11) angeordnet ist, wobei die Tragstruktur (11) eine rohrförmige Wandung bildet,
**dadurch gekennzeichnet, dass**
die seitliche Öffnung (12) der Tragstruktur (11) in einer Ebene mit der rohrförmigen Wandung angeordnet ist und somit einer Krümmung der rohrförmigen Wandung folgt, und dass die Herzklappe (15) innerhalb der seitlichen Öffnung (12) fest mit der stentartigen rohrförmigen Tragstruktur (11) verbunden ist.

2. Herzklappenprothese (10) nach Anspruch 1,
**dadurch gekennzeichnet, dass**
die Tragstruktur (11) zwei längsaxiale Endabschnitte (13) aufweist, die zur Verankerung in der oberen und unteren Hohlvene (VCS, VCI) angepasst sind.

3. Herzklappenprothese (10) nach Anspruch 2,
**dadurch gekennzeichnet, dass**
die Tragstruktur (11) einen Atriumabschnitt (14) aufweist, der sich zwischen den längsaxialen Endabschnitten (13) erstreckt.

4. Herzklappenprothese (10) nach Anspruch 3,
**dadurch gekennzeichnet, dass**
der Atriumabschnitt (14) eine Länge aufweist, die im wenigstens dem Abstand zwischen der oberen Hohlvene (VCS) und der unteren Hohlvene (VCI) entspricht.

5. Herzklappenprothese (10) nach Anspruch 3 oder 4,
**dadurch gekennzeichnet, dass**
die seitliche Öffnung (12), insbesondere die Herzklappe (15), im Atriumabschnitt (14) der Tragstruktur (11) angeordnet ist.

6. Herzklappenprothese (10) nach einem der vorhergehenden Ansprüche,
**dadurch gekennzeichnet, dass**
die Tragstruktur (11) ein Drahtgeflecht und/oder eine lasergeschnittene Gitterstruktur aufweist, die jeweils geschlossene Maschen oder Zellen umfassen.

7. Herzklappenprothese (10) nach einem der vorhergehenden Ansprüche,
**dadurch gekennzeichnet, dass**
die Tragstruktur (11) an wenigstens einem längsaxialen Ende (13) ein Durchlasssegment aufweist, das frei von der Umhüllung ist.

8. Herzklappenprothese (10) nach Anspruch 7,
**dadurch gekennzeichnet, dass**
das Durchlasssegment Maschen mit einer Maschenweite aufweist, die größer als die Maschenweite anderer Maschen der Tragstruktur (11) ist.

9. Herzklappenprothese (10) nach einem der vorhergehenden Ansprüche,
**dadurch gekennzeichnet, dass**
sich die Umhüllung (16) fluiddicht an die biologische Herzklappe (15) anschließt.

10. Herzklappenprothese (10) nach einem der vorhergehenden Ansprüche,
**dadurch gekennzeichnet, dass**
die Tragstruktur (11) ein Formgedächtnismaterial und/oder superelastisches Material, insbesondere eine Nickel-Titan-Legierung, vorzugsweise Nitinol, aufweist.

11. Herzklappenprothese (10) nach einem der vorhergehenden Ansprüche,
**dadurch gekennzeichnet, dass**
die Herzklappenprothese (10) transluminal, insbesondere transfemoral, implantierbar ist.

12. System mit einer Herzklappenprothese (10) nach einem der vorhergehenden Ansprüche und einem Katheter, wobei die Herzklappenprothese (10) in einem komprimierten Zustand innerhalb des Katheters positionierbar und mittels des Katheters perkutan, insbesondere transluminal, vorzugsweise transfemoral, implantierbar ist.

## Claims

1. A heart valve prosthesis (10), for the treatment of an insufficient tricuspid valve (TPC), the heart valve prosthesis (10) comprising a stent-type tubular support structure (11) and a biological heart valve (15), wherein the stent-type support structure (11) is configured to autonomously expand from a radially compressed state into a radially expanded state and has a fluid-tight tubular covering (16) tubularly extending over at least portions of the stent-type support structure, wherein the heart valve (15) is disposed in a lateral opening (12) of the support structure (11), wherein the support structure (11) forms a tubular wall ,
**characterized in that**
the lateral opening (12) of the support structure (11) is disposed in a common plane with the tubular wall, and, thus, follows a curvature of the tubular wall, and that the heart valve (15) is fixedly connected with the stent-type tubular support structure (11) within the lateral opening (12).

2. The heart valve prosthesis (10) of claim 1,
**characterized in that**
the support structure (11) comprises two axially longitudinal end sections (13) adapted respectively to anchoring in the superior vena cava (VCS) and inferior vena cava (VCI).

3. The heart valve prosthesis (10) of claim 2,
**characterized in that**
the support structure (11) comprises an atrium section (14) that extends between the axially longitudinal end sections (13).

4. The heart valve prosthesis of claim 3,
**characterized in that**
the atrium section (14) is of a length that corresponds to at least a distance between the superior vena cava (VCS) and the inferior vena cava (VCI).

5. The heart valve prosthesis (10) of claim 3 or 4,
**characterized in that**
the lateral opening (12), in particular the heart valve (15), is arranged in the atrium section (14) of the support structure (11).

6. The heart valve prosthesis (10) of any of the preceding claims,
**characterized in that**
the support structure (11) comprises a wire mesh and/or laser-cut lattice structure respectively enclosing closed meshes or cells.

7. The heart valve prosthesis (10) of any of the preceding claims,
**characterized in that**
the support structure (11) comprises a pass-through segment without any tubular covering disposed at least at one axially longitudinal end (13).

8. The heart valve prosthesis (10) of claim 7,
**characterized in that**
the pass-through segment comprises meshes of a larger mesh size than a mesh size of other meshes of the stent-type support structure (11).

9. The heart valve prosthesis (10) of any of the preceding claims,
**characterized in that**
the tubular covering (16) is attached in a fluid-tight manner to the biological heart valve (15).

10. The heart valve prosthesis of any of the preceding claims,
**characterized in that**
the stent-type support structure (11) comprises a shape-memory material and/or a superelastic material, in particular a nickel-titanium alloy, preferably Nitinol.

11. The heart valve prosthesis (10) of any of the preceding claims,
**characterized in that**
the heart valve prosthesis is implantable transluminally, in particularly transfemorally.

12. System having a heart valve prosthesis (10) according to any of the preceding claims and a catheter, wherein the heart valve prosthesis (10) is positionable within the catheter in a compressed stated, and is percutaneously implantable by the catheter, in particular transluminally, preferably transfemorally.

## Revendications

1. Prothèse de valvule cardiaque (10) pour le traitement d'une valvule tricuspide (TPK) insuffisante comprenant une structure de support de forme tubulaire de type stent (11) et une valvule cardiaque biologique (15), la structure porteuse de type stent (11) pouvant être automatiquement déployée d'un état comprimé radialement à un état déployé radialement et présentant une enveloppe étanche aux fluides (16) qui s'étend au moins en partie sous forme tubulaire par-dessus la structure porteuse (11), la valvule cardiaque (15) étant disposée dans une ouverture latérale (12) de la structure porteuse (11), la structure porteuse (11) formant une paroi tubulaire,
**caractérisée en ce que**
l'ouverture latérale (12) de la structure porteuse (11) est disposée dans un plan avec la paroi de forme tubulaire et suit ainsi une courbure de la paroi de forme tubulaire, et **en ce que** la valvule cardiaque (15) est connectée fixement à la structure porteuse de forme tubulaire de type stent (11) à l'intérieur de l'ouverture latérale (12).

2. Prothèse de valvule cardiaque (10) selon la revendication 1,
**caractérisée en ce que**
la structure porteuse (11) présente deux portions d'extrémité axiales longitudinales (13) qui sont adaptées à un ancrage dans la veine cave supérieure et la veine cave inférieure (VCS, VCI).

3. Prothèse de valvule cardiaque (10) selon la revendication 2,
**caractérisée en ce que**
la structure porteuse (11) présente une portion d'atrium (14) qui s'étend entre les portions d'extrémité axiales longitudinales (13).

4. Prothèse de valvule cardiaque (10) selon la revendication 3,
**caractérisée en ce que**
la portion d'atrium (14) présente une longueur qui correspond au moins à la distance entre la veine cave supérieure (VCS) et la veine cave inférieure (VCI).

5. Prothèse de valvule cardiaque (10) selon la revendication 3 ou 4,
**caractérisée en ce que**
l'ouverture latérale (12), en particulier la valvule cardiaque (15), est disposée dans la portion d'atrium (14) de la structure porteuse (11).

6. Prothèse de valvule cardiaque (10) selon l'une quelconque des revendications précédentes,
**caractérisée en ce que**
la structure porteuse (11) présente un treillis de fil métallique et/ou une structure en grillage découpée au laser, qui comprennent dans chaque cas des mailles ou des cellules fermées.

7. Prothèse de valvule cardiaque (10) selon l'une quelconque des revendications précédentes,
**caractérisée en ce que**
la structure porteuse (11) présente, au niveau d'au moins une extrémité axiale longitudinale (13), un segment de passage qui est exempt de l'enveloppe.

8. Prothèse de valvule cardiaque (10) selon la revendication 7,
**caractérisée en ce que**
le segment de passage présente des mailles ayant une largeur de maille qui est supérieure à la largeur de maille d'autres mailles de la structure porteuse (11).

9. Prothèse de valvule cardiaque (10) selon l'une quelconque des revendications précédentes,
**caractérisée en ce que**
l'enveloppe (16) se raccorde de manière étanche aux fluides à la valvule cardiaque biologique (15).

10. Prothèse de valvule cardiaque (10) selon l'une quelconque des revendications précédentes,
**caractérisée en ce que**
la structure porteuse (11) présente un matériau à mémoire de forme et/ou un matériau super élastique, en particulier un alliage de nickel-titane, de préférence du nitinol.

11. Prothèse de valvule cardiaque (10) selon l'une quelconque des revendications précédentes,
**caractérisée en ce que**
la prothèse de valvule cardiaque (10) peut être implantée par voie transluminale, en particulier transfémorale.

12. Système comprenant une prothèse de valvule cardiaque (10) selon l'une quelconque des revendications précédentes et un cathéter, la prothèse de valvule cardiaque (10) pouvant être positionnée dans un état comprimé à l'intérieur du cathéter et pouvant être implantée par voie percutanée, notamment transluminale, de préférence transfémorale, au moyen du cathéter.
